# EUROPEAN PATENT APPLICATION

(11) **EP 4 180 062 A2**
(43) Date of publication of application: **17.05.2023**
(21) Application number: 22206793.6
(22) Date of filing: 10.11.2022
(51) Int. Cl.: A61L 2/00, A47K 10/48, A61L 2/10, B64D 11/02

(54) **SYSTEMS AND METHODS FOR DISINFECTING HANDS**

(30) Priority: 11.11.2021 US 202117523951
(71) Applicant: The Boeing Company, Arlington, VA 22202 (US)
(72) Inventor: CHILDRESS, Jamie J., CHICAGO, 60606-1596 (US); KING, Teresa A., CHICAGO, 60606-1596 (US)
(74) Representative: Arnold & Siedsma

(57) **Abstract**

A system (100) for disinfecting hands (120) includes one or more ultraviolet (UV) light emitters configured to emit UV light into a hand disinfection area (122), and one or more reflectors (104) configured to reflect the UV light within the hand disinfection area (122). In at least one example, the system (100) also includes a dryer (108) configured to emit drying air into the hand disinfection area (122).

## Description

### BACKGROUND OF THE DISCLOSURE

Lavatories typically include a faucet over a sink so that individuals can wash their hands. Additionally, hand dryers blow warm or hot air onto hands after washing in order to dry moisture therefrom.

However, water spray from hand dryers can contain pathogens. Such pathogens can land on surfaces proximate to hand dryers.

Moreover, activating a hand dryer draws power. In various circumstances and settings, power can be limited, such as within an internal cabin of a commercial aircraft.

### SUMMARY OF THE DISCLOSURE

A need exists for an improved system and method for drying hands. Further, a need exists for a system and a method for disinfecting hands and/or moisture during a hand drying process.

With those needs in mind, certain examples of the present disclosure provide a system for disinfecting hands. The system includes one or more ultraviolet (UV) light emitters configured to emit UV light into a hand disinfection area, and one or more reflectors configured to reflect the UV light within the hand disinfection area.

In at least one example, the system also includes a dryer configured to emit drying air into the hand disinfection area.

In at least one example, the one or more UV light emitters are configured to emit the UV light between 215-235 nanometers.

In at least one example, the one or more reflectors are formed of one or both of Teflon or Aluminum.

In at least one example, the one or more UV light emitters include a first UV light emitter and a second UV light emitter. In at least one example, the one or more reflectors include a first reflector and a second reflector.

In at least one example, the system also includes one or more sensors configured to detect presence of the hands within the hand disinfection area.

In at least one example, a control unit is configured to control operation of the one or more UV light emitters. The control unit can be configured to continue operating the one or more UV light emitters for a predetermined period of time after the hands have been removed from hand disinfection area. The control unit can further be configured to disable or reduce power to one or more other powered systems when the one or more UV light emitters emit the UV light into the hand disinfection area.

Certain examples of the present disclosure provide a method for disinfecting hands. The method includes emitting, by one or more ultraviolet (UV) light emitters, UV light into a hand disinfection area; and reflecting, by one or more reflectors, the UV light within the hand disinfection area. In at least one example, the method also includes emitting, by a dryer, drying air into the hand disinfection area.

Certain examples of the present disclosure provide a vehicle including an internal cabin including a lavatory. The lavatory includes a system for disinfecting hands, as described herein.

### BRIEF DESCRIPTION OF THE DRAWINGS

Figure 1 illustrates a schematic block diagram of a system configured to disinfect hands.
Figure 2 illustrates a schematic diagram of the system configured to disinfect hands.
Figure 3 illustrates a schematic diagram of the system configured to disinfect hands within a lavatory.
Figure 4 illustrates a perspective front view of an aircraft.
Figure 5 illustrates a perspective internal view of a lavatory.
Figure 6 illustrates a flow chart of a method for disinfecting hands.

### DETAILED DESCRIPTION OF THE DISCLOSURE

The foregoing summary, as well as the following detailed description of certain examples will be better understood when read in conjunction with the appended drawings. As used herein, an element or step recited in the singular and preceded by the word "a" or "an" should be understood as not necessarily excluding the plural of the elements or steps. Further, references to "one example" are not intended to be interpreted as excluding the existence of additional examples that also incorporate the recited features. Moreover, unless explicitly stated to the contrary, examples "comprising" or "having" an element or a plurality of elements having a particular condition can include additional elements not having that condition.

Examples of the present disclosure provide a system and a method for drying and disinfecting hands. The drying and disinfecting can occur simultaneously. In at least one example, an ultraviolet (UV) light augmented hand dryer includes one or more UV light emitters (such as can emit UV light in the far UV spectrum, such as at 222 nanometers (nm)), and a hand dryer, which can be activated and deactivated in a touchless manner. The UV light emitter(s) is configured to illuminate to both disinfect the hands and the water droplets that spray off the hands. In at least one example, the dryer includes multiple UV light emitters and multiple reflectors to improve coverage of the UV light in relation to the hands and water spray.

The UV emitters and reflectors eliminate, minimize, or otherwise reduce pathogens within spray, such as by neutralizing (for example, killing) the pathogens in the air and/or on surfaces. The reflectors are configured to reflect the UV light onto hands between the emitters the reflectors.

In at least one example, the UV light emitters are activated in response to hands being detected within a disinfection area, such as between the UV light emitters, the reflectors, and a dryer. As a further example, the UV light emitters remain active to emit UV light after air ceases being emitted by the dryer, such as for a predetermined time to increase disinfection of surfaces. Other powered systems (UV or otherwise) in a lavatory may also deactivated (and/or power reduced thereto) when the UV light emitters are activated to reduce total power draw.

Certain examples of the present disclosure provide an automatic hand dryer and UV disinfection system configured to dry and clean hands. The system includes one or more reflector(s) to prevent shadowing of UV light on hands during a disinfection process. The system can be configured to notify completion of a disinfection process based on time, sensed distance to hands, sensed movement of hands (via infrared or optical sensor), and/or the like.

In at least one example, the system includes a housing having the reflector(s). In at least one example, the dryer and the UV light emitter(s) are separately controlled (for example, the UV light emitter(s) can be deactivated, as the dryer remain activated, or vice versa). Alternatively, users can choose whether to use air dryer or the UV lights, such as via button or gesture control. In another example, no air dryer is necessary. The system can also include a sensor configured to detect moisture on hands and control drying and/or disinfection accordingly.

In at least one example, if a touchless faucet is used, the system can activate the dryer automatically when hands are inserted into the housing. A gray use water grill can be provided at the bottom of the housing to capture gray water that drips from the hands.

As described herein, examples of the present disclosure provide systems and method that reduce the spread of pathogens within an environment, such as within an internal cabin of a vehicle.

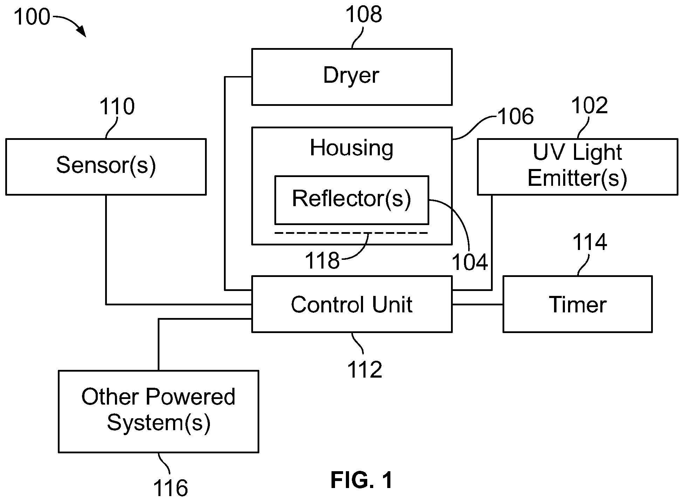

Figure 1 illustrates a schematic block diagram of a system 100 configured to disinfect hands, according to an example of the present disclosure. The system 100 includes one or more UV light emitters 102 configured to emit UV light onto one or more reflectors 104. The reflectors 104 can be disposed on or within a housing 106. Optionally, the reflectors 104 can be mounted or otherwise secured on surfaces without the use of a separate housing.

In at least one example, the UV light emitters 102 are configured to emit the UV light within the far UV spectrum. As an example, the UV light emitters 102 are configured to emit UV light within a spectrum between 215 nanometers (nm) and 235 nm. As a further example, the UV light emitters 102 are configured to emit the UV light at a wavelength of 222 nm.

In at least one example, the reflector(s) 104 can be circular or oval shaped. Optionally, the reflectors 104 can be shaped differently, such as rectangular. In at least one example, the reflectors 104 can be tubular, and configured to receive hands within a central passage. The reflector(s) 104 can be supported by a frame. The reflector(s) 104 can be large enough for 360 degrees of reflection onto hands.

The reflector(s) 104 are configured to reflect the UV light. In at least one example, the reflector(s) 104 are formed of Teflon. As a further example, the reflector(s) 104 are formed of PMR 10, which has been found to be more than 90% reflective of UV light at 222 nm. As another example, the reflector(s) 104 are formed of Aluminum. Optionally, the reflector(s) 104 can be formed of various other materials that are configured to reflect UV light.

In at least one example, a first UV light emitter 102 and a second UV light emitter 102 are configured to emit the UV light onto a first reflector 104, such as below a hand disinfection area (that is, where an individual positions hands), and a second reflector 104 above the hand disinfection area. The UV light emitters 102 emit the UV light onto the first reflector 104, which reflects the UV light into the hand disinfection area and toward the second reflector 104, which reflects the UV light back toward the hand disinfection area and the first reflector 104, thereby ensuring that opposite sides of the hands are illuminated and disinfected by the UV light (thereby reducing shadowing of the hands). The UV light emitter(s) 102 and the reflector(s) 104 eliminate, minimize, or otherwise reduce pathogens on the hands, and within spray and air, such as by neutralizing (for example, killing) the pathogens.

The system 100 also includes a dryer 108, which is configured to emit air (such as warm or hot air) into the hand disinfection area to dry the hands. The dryer 108 can be disposed above, below, in front, or behind the hand disinfection area. One of the reflectors 104 can be proximate an outlet of the dryer 108, and another of the reflectors 104 can be below (in front of, behind, or the like) the hand disinfection area, which is below (or in front of, or behind) the outlet of the dryer 108. Alternatively, the system 100 may not include the dryer 108.

The system 100 also includes one or more sensors 110, such as can be configured to detect presence of hands within the hand disinfection area. The sensor(s) 110 can be an infrared sensor, an ultrasonic sensor, another type of optical sensor, and/or the like that are configured to detect presence of the hands within the hand disinfection area. Alternatively, the system 100 may not include the sensors 110.

The system 100 also includes a control unit 112 that is configured to control operation. The control unit 112 is in communication with the UV light emitters 102, the dryer 108, and/or the sensor 110, such as through one or more wired or wireless connections. In at least one example, the control unit 112 can also be in communication with a timer 114, such as through one or more wired or wireless connections. In at least one example, the control unit 112 includes the timer 114. Optionally, the system 100 does not include the timer 114.

In operation, an individual positions hands within the hand disinfection area, such as is between the UV light emitter(s) 102, the dryer 108, and the reflectors 104. The sensor(s) 110 detects the presence of the hands within the hand disinfection area and outputs a detection signal to the control unit 112. In response to receiving the detection signal from the sensor(s) 110, the control unit 112 activates the UV light emitter(s) 102 to emit the UV light, which is reflected by the reflector(s) 104 to ensure that different portions of the hands (such as different sides) of the hands are illuminated by the UV light, thereby eliminating, minimizing, or otherwise reducing shadowing of the hands. In this manner, the reflectors 104 ensure that all or most portions of the hands are effectively disinfected by the UV light, which also disinfects moisture and area within the hand disinfection area.

The control unit 112 also activates the dryer 108 when the hands are detected within the hand disinfection area. Optionally, an individual can select only activation of the dryer, or the UV light emitter(s) 102, such as via a user interface, touchless gesture operation (as recognized by the sensor(s) 110 and/or the control unit 112, and/or the like.

In at least one example, the control unit 112 deactivates the dryer 108 in response to the sensor(s) 110 no longer detecting the presence of hands within the hand disinfection area. For example, the sensor(s) 110 output a non-detection signal to the control unit 112 in response to the hands being removed from the hand disinfection area. The control unit 112 can continue to operate the UV light emitter(s) 102 to emit the UV light into the hand disinfection area after the hands are removed for a predetermined time (such as 5 seconds or less) to disinfect surfaces surrounding the hand disinfection area (such as countertops, a sink, or the like). The timer 114 can be programmed to provide the predetermined time. In at least one example, the dryer 108 and the UV light emitter(s) 102 can be simultaneously active.

In at least one example, the control unit 112 is in communication with one or more other powered systems 116, such as through one or more wired or wireless connections. The other powered system 116 can include a water heating system, powered actuators of a toilet seat, touchless sensors of a faucet, certain illumination systems, and/or the like. During operation of the UV light emitter(s) 102, the control unit 112 can disable the other powered system(s) 116, or otherwise reduce power provided thereto. In this manner, the control unit 112 is configured to conserve overall power usage during operation of the UV light emitter(s) 102. Optionally, the control unit 112 is not in communication with the other powered systems.

In at least one example, such as if a touchless faucet is used, the system 100 can activate the dryer 108 automatically when hands are inserted into the hand disinfection area. A gray use water grill 118 can be provided at the bottom of the housing 106 to capture gray water that drips from the hands. Optionally, the system 100 may not include the gray use water grill.

As described herein, the system 100 for disinfecting hands includes the one or more ultraviolet light emitters 102 configured to emit UV light into a hand disinfection area. The one or more reflectors 104 are configured to reflect the UV light within the hand disinfection area. In at least one example, the system 100 also includes the dryer 108 configured to emit drying air into the hand disinfection area.

As used herein, the term "control unit," "central processing unit," "CPU," "computer," or the like may include any processor-based or microprocessor-based system including systems using microcontrollers, reduced instruction set computers (RISC), application specific integrated circuits (ASICs), logic circuits, and any other circuit or processor including hardware, software, or a combination thereof capable of executing the functions described herein. Such are exemplary only, and are thus not intended to limit in any way the definition and/or meaning of such terms. For example, the control unit 112 may be or include one or more processors that are configured to control operation, as described herein.

The control unit 112 is configured to execute a set of instructions that are stored in one or more data storage units or elements (such as one or more memories), in order to process data. For example, the control unit 112 may include or be coupled to one or more memories. The data storage units may also store data or other information as desired or needed. The data storage units may be in the form of an information source or a physical memory element within a processing machine.

The set of instructions may include various commands that instruct the control unit 112 as a processing machine to perform specific operations such as the methods and processes of the various examples of the subject matter described herein. The set of instructions may be in the form of a software program. The software may be in various forms such as system software or application software. Further, the software may be in the form of a collection of separate programs, a program subset within a larger program, or a portion of a program. The software may also include modular programming in the form of object-oriented programming. The processing of input data by the processing machine may be in response to user commands, or in response to results of previous processing, or in response to a request made by another processing machine.

The diagrams of examples herein may illustrate one or more control or processing units, such as the control unit 112. It is to be understood that the processing or control units may represent circuits, circuitry, or portions thereof that may be implemented as hardware with associated instructions (e.g., software stored on a tangible and non-transitory computer readable storage medium, such as a computer hard drive, ROM, RAM, or the like) that perform the operations described herein. The hardware may include state machine circuitry hardwired to perform the functions described herein. Optionally, the hardware may include electronic circuits that include and/or are connected to one or more logic-based devices, such as microprocessors, processors, controllers, or the like. Optionally, the control unit 112 may represent processing circuitry such as one or more of a field programmable gate array (FPGA), application specific integrated circuit (ASIC), microprocessor(s), and/or the like. The circuits in various examples may be configured to execute one or more algorithms to perform functions described herein. The one or more algorithms may include aspects of examples disclosed herein, whether or not expressly identified in a flowchart or a method.

As used herein, the terms "software" and "firmware" are interchangeable, and include any computer program stored in a data storage unit (for example, one or more memories) for execution by a computer, including RAM memory, ROM memory, EPROM memory, EEPROM memory, and non-volatile RAM (NVRAM) memory. The above data storage unit types are exemplary only, and are thus not limiting as to the types of memory usable for storage of a computer program.

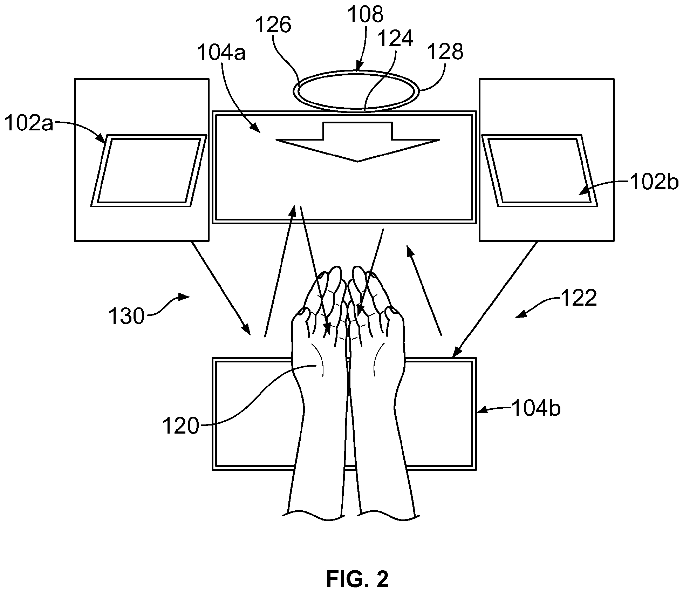

Figure 2 illustrates a schematic diagram of the system 100 configured to disinfect hands 120, according to an example of the present disclosure. As described herein, the hands 120 are positioned within the hand disinfection area 122, which is between a first UV light emitter 102a, a second UV light emitter 102b, a first reflector 104a, and a second reflector 104b. The first reflector 104a is proximate to one or more air nozzles 124 of the dryer 108. For example, the first reflector 104a is disposed around the air nozzles 124 above the hand disinfection area 122. The second reflector 104b is disposed below the hand disinfection area 122, such as on a surface of a countertop, a sink, and/or the like.

The first UV light emitter 102a is spaced from a first side 126 of the dryer 108, and the second UV light emitter 102b is spaced from a second side 128 (opposite from the first side 126) of the dryer 108. The first UV light emitter 102a and the second UV light emitter 102b are angled to emit the UV light 130 toward and onto the reflector 104b, so that the UV light 130 reflects back and forth between the reflectors 104a and 104b, thereby illuminating different surfaces of the hands 120, thereby eliminating, minimizing, or otherwise reducing shadowing effects.

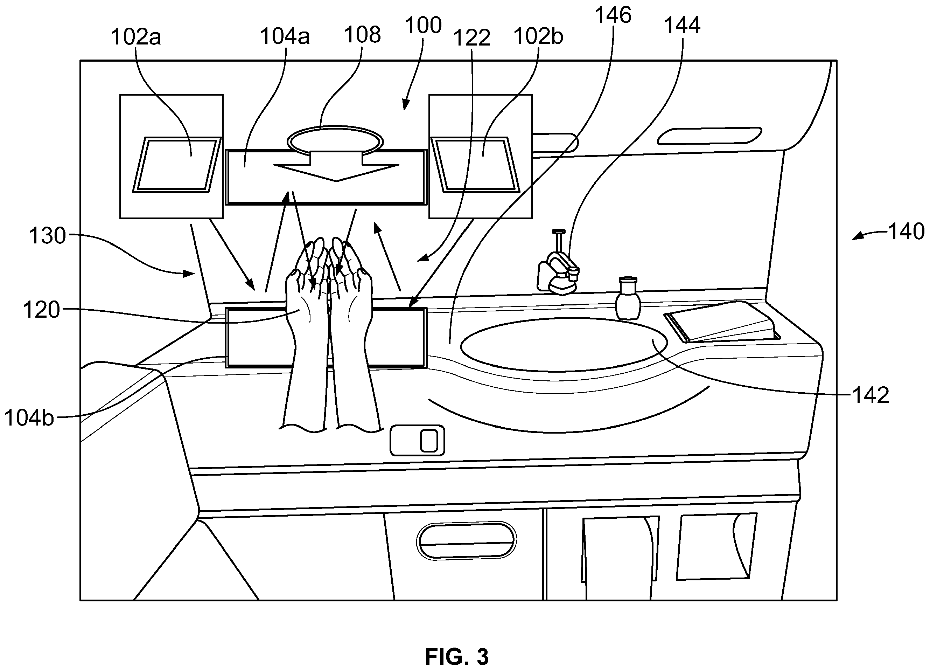

Figure 3 illustrates a schematic diagram of the system 100 configured to disinfect hands within a lavatory 140, according to an example of the present disclosure. The lavatory 140 includes a sink 142 and a faucet 144, for example. In at least one example, the lavatory 140 is within an internal cabin of a vehicle, such as a commercial aircraft, as shown, the reflector 104b can be disposed over a countertop 146 adjacent to the sink 142.

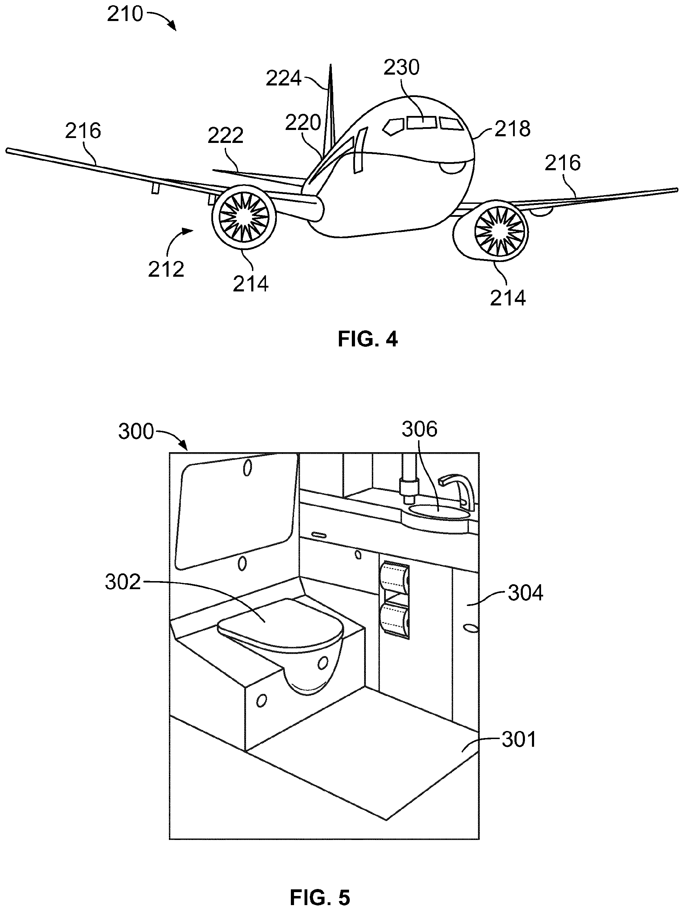

Figure 4 illustrates a perspective front view of an aircraft 210, according to an example of the present disclosure. The aircraft 210 includes a propulsion system 212 that includes engines 214, for example. Optionally, the propulsion system 212 may include more engines 214 than shown. The engines 214 are carried by wings 216 of the aircraft 210. In other embodiments, the engines 214 may be carried by a fuselage 218 and/or an empennage 220. The empennage 220 may also support horizontal stabilizers 222 and a vertical stabilizer 224.

The fuselage 218 of the aircraft 210 defines an internal cabin 230, which includes a flight deck or cockpit, one or more work sections (for example, galleys, personnel carry-on baggage areas, and the like), one or more passenger sections (for example, first class, business class, and coach sections), one or more lavatories, and/or the like. The systems and methods described herein can be used within the internal cabin 230, such as within a lavatory.

Alternatively, instead of an aircraft, examples of the present disclosure may be used with various other vehicles, such as automobiles, buses, locomotives and train cars, watercraft, and the like. Further, examples of the present disclosure may be used with respect to fixed structures, such as commercial and residential buildings.

Figure 5 illustrates a perspective internal view of a lavatory 300, according to an example of the present disclosure. The lavatory 300 is an example of an enclosed space or chamber, such as within the internal cabin 230 of the aircraft 210, shown in Figure 4. The lavatory 300 includes a door leading therein. The system 100 shown and described with respect to Figures 1-3 can be used within the lavatory 300. The lavatory 300 may be onboard an aircraft, as described above. Optionally, the lavatory 300 may be onboard various other vehicles. In other examples, the lavatory 300 may be within a fixed structure, such as a commercial or residential building. The lavatory 300 includes a base floor 301 that supports a toilet 302, one or more cabinets 304, and a sink 306 or wash basin. The lavatory 300 may be arranged differently than shown. The lavatory 300 may include more or less components than shown.

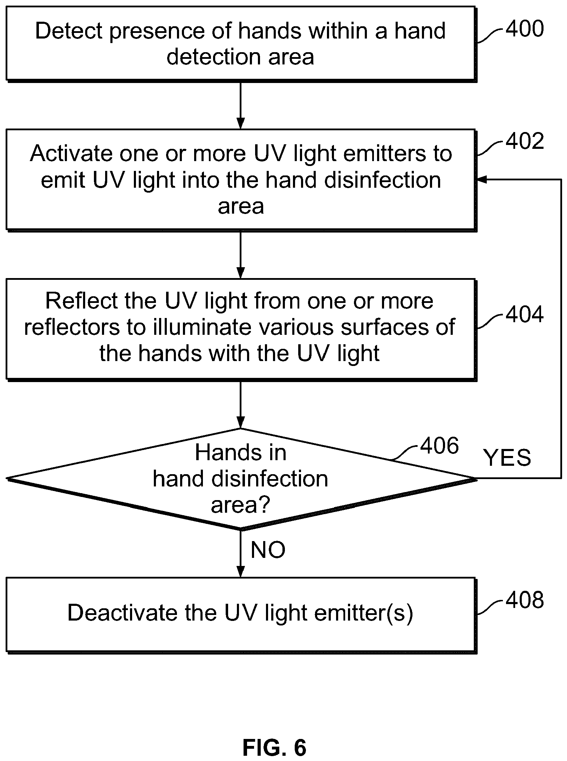

Figure 6 illustrates a flow chart of a method for disinfecting hands, according to an example of the present disclosure. Referring to Figures 1-3 and 6, at 400, a presence of hands 120 are detected within the hand detection area 122, such as by the sensor(s) 110. In response, at 402, the UV light emitter(s) 102 are activated, such as by the control unit 112, to emit the UV light 130 into the hand disinfection area 122. The dryer 108 can optionally be activated, as well. At 404, the UV light 130 is reflected from the one or more reflectors 104 to illuminate various surface of the hands 120 with the UV light 130. At 406, the control unit 112 determines if the hands 120 are still in the hand disinfection area 122. If so, the process returns to 402, at which the UV light emitter(s) 102 remain activated. If, however, the hands are no longer detected within the hand disinfection area at 406, the method proceeds to 408, at which the UV light emitter(s) 102 are deactivated, such as after a predetermined time.

Further, the disclosure comprises examples according to the following clauses:
Clause 1. A system for disinfecting hands, the system comprising:
   one or more ultraviolet (UV) light emitters configured to emit UV light into a hand disinfection area; and
   one or more reflectors configured to reflect the UV light within the hand disinfection area.
Clause 2. The system of Clause 1, further comprising a dryer configured to emit drying air into the hand disinfection area.
Clause 3. The system of Clauses 1 or 2, wherein the one or more UV light emitters are configured to emit the UV light between 215-235 nanometers.
Clause 4. The system of any of Clauses 1-3, wherein the one or more reflectors are formed of one or both of Teflon or Aluminum.
Clause 5. The system of any of Clauses 1-4, wherein the one or more UV light emitters comprise a first UV light emitter and a second UV light emitter.
Clause 6. The system of any of Clauses 1-5, wherein the one or more reflectors comprise a first reflector and a second reflector.
Clause 7. The system of any of Clauses 1-6, further comprising one or more sensors configured to detect presence of the hands within the hand disinfection area.
Clause 8. The system of any of Clauses 1-7, further comprising a control unit configured to control operation of the one or more UV light emitters.
Clause 9. The system of Clause 8, wherein the control unit is configured to continue operating the one or more UV light emitters for a predetermined period of time after the hands have been removed from hand disinfection area.
Clause 10. The system of Clauses 8 or 9, wherein the control unit is further configured to disable or reduce power to one or more other powered systems when the one or more UV light emitters emit the UV light into the hand disinfection area.
Clause 11. A method for disinfecting hands, the method comprising:
   emitting, by one or more ultraviolet (UV) light emitters, UV light into a hand disinfection area; and
   reflecting, by one or more reflectors, the UV light within the hand disinfection area.
Clause 12. The method of Clause 11, further comprising emitting, by a dryer, drying air into the hand disinfection area.
Clause 13. The method of Clauses 11 or 12, wherein the one or more UV light emitters are configured to emit the UV light between 215-235 nanometers.
Clause 14. The method of any of Clauses 11-13, wherein the one or more reflectors are formed of one or both of Teflon or Aluminum.
Clause 15. The method of any of Clauses 11-14, wherein the one or more UV light emitters comprise a first UV light emitter and a second UV light emitter, and wherein the one or more reflectors comprise a first reflector and a second reflector.
Clause 16. The method of any of Clauses 11-15, further comprising detecting, by one or more sensors, presence of the hands within the hand disinfection area, and wherein said emitting occurs in response to said detecting.
Clause 17. The method of any of Clauses 11-16, further comprising controlling, by a control unit, operation of the one or more UV light emitters.
Clause 18. The method of any of Clauses 11-17, further comprising continuing said emitting for a predetermined period of time after the hands have been removed from hand disinfection area.
Clause 19. The method of any of Clauses 11-18, further comprising disabling or reducing power to one or more other powered systems during said emitting.
Clause 20. A vehicle comprising:
   an internal cabin including a lavatory, wherein the lavatory comprises a system for disinfecting hands, the system comprising:
   one or more ultraviolet (UV) light emitters configured to emit UV light into a hand disinfection area, wherein the one or more UV light emitters are configured to emit the UV light between 215-235 nanometers;
   one or more reflectors configured to reflect the UV light within the hand disinfection area, wherein the one or more reflectors are formed of one or both of Teflon or Aluminum;
   a dryer configured to emit drying air into the hand disinfection area;
   one or more sensors configured to detect presence of the hands within the hand disinfection area; and
   a control unit configured to control operation of the one or more UV light emitters, wherein the control unit is configured to continue operating the one or more UV light emitters for a predetermined period of time after the hands have been removed from hand disinfection area, and wherein the control unit is further configured to disable or reduce power to one or more other powered systems when the one or more UV light emitters emit the UV light into the hand disinfection area.

As described herein, examples of the present disclosure provide improved systems and methods for drying hands. Further, examples of the present disclosure provide systems and methods for disinfecting hands and/or moisture during a hand drying process.

While various spatial and directional terms, such as top, bottom, lower, mid, lateral, horizontal, vertical, front and the like can be used to describe examples of the present disclosure, it is understood that such terms are merely used with respect to the orientations shown in the drawings. The orientations can be inverted, rotated, or otherwise changed, such that an upper portion is a lower portion, and vice versa, horizontal becomes vertical, and the like.

As used herein, a structure, limitation, or element that is "configured to" perform a task or operation is particularly structurally formed, constructed, or adapted in a manner corresponding to the task or operation. For purposes of clarity and the avoidance of doubt, an object that is merely capable of being modified to perform the task or operation is not "configured to" perform the task or operation as used herein.

It is to be understood that the above description is intended to be illustrative, and not restrictive. For example, the above-described examples (and/or aspects thereof) can be used in combination with each other. In addition, many modifications can be made to adapt a particular situation or material to the teachings of the various examples of the disclosure without departing from their scope. While the dimensions and types of materials described herein are intended to define the parameters of the various examples of the disclosure, the examples are by no means limiting and are exemplary examples. Many other examples will be apparent to those of skill in the art upon reviewing the above description. The scope of the various examples of the disclosure should, therefore, be determined with reference to the appended claims, along with the full scope of equivalents to which such claims are entitled. In the appended claims and the detailed description herein, the terms "including" and "in which" are used as the plain-English equivalents of the respective terms "comprising" and "wherein." Moreover, the terms "first," "second," and "third," etc. are used merely as labels, and are not intended to impose numerical requirements on their objects. This written description uses examples to disclose the various examples of the disclosure, including the best mode, and also to enable any person skilled in the art to practice the various examples of the disclosure, including making and using any devices or systems and performing any incorporated methods. The patentable scope of the various examples of the disclosure is defined by the claims, and can include other examples that occur to those skilled in the art. Such other examples are intended to be within the scope of the claims if the examples have structural elements that do not differ from the literal language of the claims, or if the examples include equivalent structural elements with insubstantial differences from the literal language of the claims.

## Claims

1. A system (100) for disinfecting hands (120), the system (100) comprising:
one or more ultraviolet (UV) light emitters configured to emit UV light into a hand disinfection area (122); and
one or more reflectors (104) configured to reflect the UV light within the hand disinfection area (122).

2. The system (100) of claim 1, further comprising a dryer (108) configured to emit drying air into the hand disinfection area (122).

3. The system (100) of claim 1 or 2, wherein the one or more UV light emitters (102) are configured to emit the UV light between 215-235 nanometers.

4. The system (100) of any of claims 1-3, wherein the one or more reflectors (104) are formed of one or both of Teflon or Aluminum.

5. The system (100) of any of claims 1-4, wherein the one or more UV light emitters (102) comprise a first UV light emitter (102) and a second UV light emitter (102).

6. The system (100) of any of claims 1-5, wherein the one or more reflectors (104) comprise a first reflector and a second reflector.

7. The system (100) of any of claims 1-6, further comprising one or more sensors (110) configured to detect presence of the hands (120) within the hand disinfection area (122).

8. The system (100) of any of claims 1-7, further comprising a control unit (112) configured to control operation of the one or more UV light emitters (102).

9. The system (100) of claim 8, wherein the control unit (112) is configured to continue operating the one or more UV light emitters (102) for a predetermined period of time after the hands (120) have been removed from hand disinfection area (122).

10. The system (100) of claim 8 or 9, wherein the control unit (112) is further configured to disable or reduce power to one or more other powered systems (116) when the one or more UV light emitters (102) emit the UV light into the hand disinfection area (122).

11. A method for disinfecting hands (120), the method comprising:
emitting, by one or more ultraviolet (UV) light emitters, UV light into a hand disinfection area (122); and
reflecting, by one or more reflectors (104), the UV light within the hand disinfection area (122).

12. The method of claim 11, further comprising emitting, by a dryer (108), drying air into the hand disinfection area (122).

13. The method of claim 11 or 12, wherein the one or more UV light emitters (102) are configured to emit the UV light between 215-235 nanometers.

14. The method of any of claims 11-13, further comprising detecting, by one or more sensors (110), presence of the hands (120) within the hand disinfection area (122), and wherein said emitting occurs in response to said detecting.

15. A vehicle comprising:
an internal cabin including a lavatory (140), wherein the lavatory (140) comprises a system (100) for disinfecting hands (120), the system (100) comprising:
one or more ultraviolet (UV) light emitters configured to emit UV light into a hand disinfection area (122), wherein the one or more UV light emitters (102) are configured to emit the UV light between 215-235 nanometers;
one or more reflectors (104) configured to reflect the UV light within the hand disinfection area (122), wherein the one or more reflectors (104) are formed of one or both of Teflon or Aluminum;
a dryer (108) configured to emit drying air into the hand disinfection area (122);
one or more sensors (110) configured to detect presence of the hands (120) within the hand disinfection area (122); and
a control unit (112) configured to control operation of the one or more UV light emitters (102), wherein the control unit (112) is configured to continue operating the one or more UV light emitters (102) for a predetermined period of time after the hands (120) have been removed from hand disinfection area (122), and wherein the control unit (112) is further configured to disable or reduce power to one or more other powered systems (116) when the one or more UV light emitters (102) emit the UV light into the hand disinfection area (122).
